Europäisches Patentamt

European Patent Office

Office européen des brevets

(11) Veröffentlichungsnummer: **0 297 291 B1**

(19)

(12) **EUROPÄISCHE PATENTSCHRIFT**

(45) Veröffentlichungstag der Patentschrift: **06.10.93**

(21) Anmeldenummer: **88108609.4**

(22) Anmeldetag: **30.05.88**

(51) Int. Cl.⁵: **C12N 15/31, A61K 39/104, C12Q 1/68, G01N 33/577**

(54) **Äusseres Membranprotein F von Pseudomonas aeruginosa.**

(30) Priorität: **03.06.87 DE 3718591**

(43) Veröffentlichungstag der Anmeldung:
**04.01.89 Patentblatt 89/01**

(45) Bekanntmachung des Hinweises auf die
Patenterteilung:
**06.10.93 Patentblatt 93/40**

(84) Benannte Vertragsstaaten:
**AT BE CH DE ES FR GB IT LI LU NL SE**

(56) Entgegenhaltungen:

**INFECTION AND IMMUNITY, Band 44, Nr. 1, April 1984, S. 49-54; Am. Soc. for Microbiology, US; H.E. GILLELAND Jr. et al.: "Use of a purified outer membrane protein F (poin) preparation of Pseudomonas aeruginosa as a protective vaccine in mice"**

**CHEMICAL ABSTRACTS, Band 108, Nr. 25, 20. Juni 1988, S. 143, Ref. Nr. 217037a; Columbus, Ohio, US; M. DUCHENE et al.: "Sequence and transcriptional start site of the Pseudomonas aeruginosa outer membrane proin protein F gene"**

(73) Patentinhaber: **BEHRINGWERKE Aktiengesellschaft**
**Postfach 1140**
**D-35001 Marburg(DE)**

(72) Erfinder: **Domdey, Horst, Dr.**
**Fasanenweg 6**
**D-8027 Neuried(DE)**
Erfinder: **Lottspeich, Friedrich, Dr.**
**Drosselweg 1**
**D-8027 Neuried(DE)**
Erfinder: **von Specht, Bernd-Ulrich, Prof. Dr.**
**Am Waldweg**
**D-8193 Ambach(DE)**
Erfinder: **Duchene, Michael, Dr.**
**Gabelsbergerstrasse 59**
**D-8000 München 2(DE)**

EP 0 297 291 B1

EP 0 297 291 B1

INFECTION AND IMMUNITY, Band 56, Nr. 5, May 1988, S. 1017-1022; Am. Soc. for Microbiology, Washington, US; H.E. GILLELAND Jr. et al.: "Outer membrane protein F preparation of Pseudomonas aeruginosa as a vaccine against chronic pulmonary infection with heterologous immunotype strains in a rat model"

CHEMICAL ABSTRACTS, Band 103, Nr. 9, 2. September 1985, S. 493, Ref. Nr. 69460n; Columbus, Ohio, US; R.E.W. HANCOCK et al.: "Immunotherapeutic potential of monoclonal antibodies against Pseudomonas Aeruginosa protein F"

CHEMICAL ABSTRACTS, Band 103, Nr. 1, 8. Juli 1985, S. 433, Ref. Nr. 4711a; Columbus, Ohio, US; L.M. MUTHARIA et al.: "Characterization of two surface-localized antigenic sites on porin protein F of Pseudomonas aeruginosa"

CHEMICAL ABSTRACTS, Band 101, Nr. 25, 17. Dezember 1984, S. 586, Ref.Nr. 228262b; Columbus, Ohio, US, S. SAWADA et al.: "Protection against infection with Pseudomonas aeruginosa by passive transfer of monoclonal antibodies to lipopolysaccharides and outer membrane proteins"

INFECTION AND IMMUNITY, Band 54, Nr. 1, Oktober 1986, S. 239-244; Am. Soc. for Microbiology, US; J.E. PENNINGTON et al.: "Polyclonal and monoclonal antibody therapy for experimental Pseusomonas aeruginosa pneumonia"

INFECTION AND IMMUNITY, Band 42, Nr. 3, Dezember 1983, S. 1027-1033; Am. Soc. for Microbiology, US; L.M. MUTHARIA et al.: "Surface localization of Pseudomonas aeruginosa outer membrane porin protein F by using monoclonal antibodies"

BIOLOGICAL ABSTRACTS/RRM, Ref.Nr. 25071807; Philadelphia, US; D.R. COOK et al.: "Detection of Pseudomonas aeruginosa antigen in serum using a PAN reactive monoclonal antibody"

Chemical Abstracts, Band 105, Nr. 15, 13. Okt. 1986, S. 212, Ref. Nr. 128784n; Columbus, Ohio, US, W.A. Woodruff et al.

Chemical Abstracts, Band 107, Nr. 7, 17. August 1987, S. 566, Ref. Nr. 57059h; Columbus, Ohio, US; J.M. Matthews-Greer et al.

Biological Abstracts/RRM, Ref. Nr. 27016703

**Beschreibung**

Pseudomonas aeruginosa ist ist ein ubiquitär vorkommender Mikroorganismus, der in der Humanmedizin als "Problemkeim" bekannt ist. Er befällt in erster Linie geschwächte Patienten und ist häufig durch Antibiotikatherapie nur schwer zu bekämpfen. Besonders gefährdet sind deshalb Patienten auf Intensivstationen und Querschnittsgelähmte sowie Menschen, die Verbrennungen erlitten oder der Gefahr von Verbrennungen ausgesetzt sind, wie Feuerwehrleute und Stahlarbeiter.

W.A. Woodruff et al., J. Bacteriol. 167 (1986) 473-479 beschreiben die Expression des äußeren Membranproteins F (Outer Membrane Protein F, OMPF, Porin F) in E. coli, wobei ein nicht näher definierter Stamm P. aeruginosa PAO1 als Ausgangsstamm diente. Die klonierten DNA-Sequenzen sind nur durch sehr grobe Restriktionskarten charakterisiert; es sind weder DNA-Sequenzen noch Aminosäure-Teilsequenzen angegeben und es wird auch auf keine Hinterlegung des klonierten Materials bei einer anerkannten Hinterlegungsstelle verwiesen.

Der Erfindung liegt die komplette Charakterisierung des Gens für OMPF aus P. aeruginosa, Serotyp 6, ATCC 33354, zugrunde. Die DNA-Sequenz und die daraus abgeleitete Aminosäuresequenz sind in der Tabelle wiedergegeben, wobei die Aminosäuresequenz im Einbuchstabencode unter den betreffenden Tripletts angeordnet ist und das Signalpeptid durch kursive Buchstaben hervorgehoben ist.

Hierzu wurden aus dem genannten Stamm die Proteine der äußeren Membran gewonnen und daraus mittels HPLC das OMPF angereichert. Der Aminoterminus und durch Spaltung mit Trypsin erhaltene Proteinfragmente wurden ansequenziert. Aus verschiedenen dieser Oligopeptide wurden dafür kodierende DNA-Sequenzen abgeleitet und diese Oligodesoxynukleotide chemisch synthetisiert. Weiterhin wurde aus dem Stamm genomische DNA isoliert, gereinigt und mit dem Restriktionsenzym Sau3A partiell verdaut. DNA-Fragmente von etwa 15 bis 20 kb wurden engereichert und in den λ-Phagen EMBL 3 (A.-M. Frischauf et al., J. Mol. Biol. 170 (1983) 827-842; R.W. Hendrix et al. (Eds.), Lambda II, Gold Spring Harbor Laboratory Press (1983)), kloniert. Die so erhaltene Genbank wurde mit den synthetisierten Oligodesoxynukleotiden auf komplementäre Sequenzen abgesucht. Aus einem der gefundenden positiv reagierenden Phagen-Klone wurde das Gen für OMPF auf einem 15 kb-Fragment gefunden. Auf diesem Fragment und auf weiteren gefundenen Fragmenten konnte das Gen auf einem 2,5 kb PstI-Fragment eingegrenzt werden. Es zeigte sich jedoch, daß dieses Fragment nicht in einen "high copy number"-Vektor kloniert werden konnte, da das Genprodukt offensichtlich für die Wirtszelle toxisch ist. Das Gen wurde deshalb in zwei überlappenden Teilstücken mit einem Überlappungsbereich von etwa 500 bp subkloniert. Aus den beiden Subklonen wurde die DNA-Sequenz des OMPF-Gens und der angrenzenden Bereiche bestimmt. Beide DNA-Stränge des Gens wurden vollständig (nach der Methode von Sanger) sequenziert. Aus der so erhaltenen DNA-Sequenz wurde die entsprechende Aminosäuresequenz abgeleitet. Die Aminosäuresequenzen aller Oligopeptide, die durch tryptische Spaltung des Proteins erhalten worden waren, konnten eindeutig zugeordnet werden. Das 5'-Ende der OMPF-mRNA wurde durch S1-Analyse und reverse Transkription der mRNA ermittelt.

Die Charakterisierung des Gens erlaubt nunmehr die Herstellung von OMPF und immunogenen Teilsequenzen dieses Proteins in der Menge und Reinheit, die für einen Einsatz zur Herstellung von Impfstoffen erforderlich ist.

Die Erfindung betrifft somit ein Nukleotid, kodierend für das äußere Membranprotein F (OMPF) von Pseudomonas aeruginosa, mit der DNA (kodierender Strang) gemäß Tabelle oder für immunogene Sequenzen kodierende Teile davon,Vektoren, DNA-Struktur und pro- oder eukaryotische Zellen, die die entsprechenden Nukleotidsequenzen enthalten sowie ein Verfahren zur Herstellung von OMPF oder immunogenen Teilsequenzen davon. Ein weiterer Gegenstand der Erfindung ist ein Diagnostikum, das die entsprechenden Nukleotidsequenzen enthält und Diagnostizierverfahren unter Verwendung solcher Diagnostika.

Darüber hinaus eröffnet die Erfindung einen Weg zur passiven Immunisierung mit humanen monoklonalen Antikörpern. Die Erfindung betrifft deshalb auch die Verwendung von erfindungsgemäß gewonnenen Antigenen zur Induzierung von Lymphozyten zur Produktion entsprechender monoklonaler Antikörper bzw. zum Testen von Lymphozyten auf die Produktion solcher Antikörper.

Die Aufarbeitung, Reinigung, Immunisierung und Gewinnung der Seren bzw. Antikörper kann nach an sich bekannten Methoden erfolgen. Verwiesen sei beispielsweise auf M.E. Gilleland et al., Infection and Immunity 44 No. 1 (Apr. 1984) 49-54; R.E.W. Hancock et al., J. Infectious Diseases 149 No. 2 (Feb. 1984) 220-226; S. Sawada et al., J. Infectious Diseases 150 No. 4 (Oct. 1984) 570-576.

Die Erfindung wird in den folgenden Beispielen näher erläutert. Teile und Prozentangaben beziehen sich auf das Gewicht, wenn keine anderen Angaben gemacht sind.

**Beispiel 1:**

**Gewinnung von OMPF und tryptischer Fragmente**

Die äußeren Membranproteine von Pseudomonas aeruginosa ATCC 33354 werden nach der Methode von Mizuno und Kageyama (J. Biochem. 86, 979-989, 1979) isoliert. Die Bakterienkulturen werden in der spät-logarithmischen Wachstumsphase geerntet, in 10 mM Na-Phosphat (pH 7,2; "Aufschlußpuffer") suspendiert und mit Glasperlen im Homogenisator (®Waring-Blender) bei 4°C 5 min aufgeschlossen. Die Zellwände werden durch 60-minütige Zentrifugation bei 100 000 g bei 4°C pelletiert und noch zweimal mit dem Aufschlußpuffer gewaschen. Pro 150 g Naßgewicht Pseudomonas werden zum Pellet 400 ml SDS-Puffer (2 % SDS, 10 % Glycerin, 10 mM Tris-HCl, pH 7,8) zugegeben und bei 30°C 60 min gerührt. Danach wird 60 min bei 100 000 g zentrifugiert. Der Niederschlag wird nochmals mit der halben Menge SDS-Puffer extrahiert und erneut zentrifugiert. Die lösliche Fraktion wird verworfen. Der unlösliche Niederschlag wird mit 300 ml NaCl-SDS-Puffer (2 % SDS, 10 % Glycerin, 0,1 M NaCl, 10 mM Tris-HCl, pH 7,8) 60 min bei 30°C gerührt. Anschließend wird 60 min bei 100 000 g und 25°C zentrifugiert. Das Pellet wird zweimal bei 4°C mit $H_2O$ bidest. gewaschen. Die unlösliche Fraktion enthält hauptsächlich die Proteine F und $H_2$ und kleine Mengen an Protein I.

1 mg der äußeren Membranproteine werden in 1 ml 20 % Ameisensäure/6 M Harnstoff gelöst und an TSK 3000 (LKB) in Portionen zu 150 $\mu$l in 20 % Ameisensäure chromatographiert (Flußrate 1 ml/min). Das unter diesen Bedingungen in den Proteinfraktionen erhaltene Material wird gepoolt und zeigt in der SDS-Gelelektrophorese eine einheitliche Bande, die im Molekulargewicht OMPF entspricht.

Dieses Material wird gefriergetrocknet, in 200 $\mu$l 0,1 M Tris-HCl (pH 8,0) aufgenommen und mit 20 $\mu$g (mit L-1-Tosylamid-2-phenylethyl-chlormethylketon behandeltem) Trypsin-TPCK (Cooper Biochemical GmbH) 18 Std. bei 37°C gespalten. Das Spaltgemisch wird mit Ameisensäure auf pH 3,5 gebracht und über "Reversed Phase"-Hochdruckflüssigkeitschromatographie fraktioniert.

Chromatographiebedingungen:

Säule:       ®Vydac TPRP-18 (10 $\mu$m), 250 x 4 mm (Chrompack)

Lösungsmittelsystem:

Lösungsmittel A:      0,1 % (v/v) Trifluoressigsäure (Fluka, z. Sequenzanalyse) in Wasser

Lösungsmittel B:      0,1 % (v/v) Trifluoressigsäure in Acetonitril (E. Merck, ®Lichrosolv)

Gradient von 0 % B bis 70 % B in A in 70 min

Flußrate:      1,5 ml/min

Raumtemperatur

Die erhaltenen Peptidfragmente werden zum Teil durch Aminosäureanalyse und Sequenzanalyse charakterisiert. Einige der erhaltenen Peakfraktionen werden nach Rechromatographie an TSK 2000 in 0,1 % Trifluoressigsäure auf einem Gasphasensequenzer (Applied Biosystems 470 A) analysiert. Die Phenylhydantoin-Aminosäurederivate werden mit Hilfe eines isokratischen HPLC-Systems (Lottspeich, J. Chromatography 326, 321-327, 1985) identifiziert.

**Beispiel 2:**

**Gewinnung und Charakterisierung der OMPF-DNA**

Konstruktion einer Genbank von Pseudomonas aeruginosa:

λ EMBL3-Arme werden nach Maniatis et al. (Molecular Cloning, Cold Spring Harbor Publications 1982) präpariert. P. aeruginosa-DNA wird aus einer 500 ml Kultur isoliert (Rodriguez and Tait, Recombinant DNA Techniques, Addison-Wesley, 1983). 20 x 10 $\mu$g DNA werden mit dem Restriktionsenzym Sau3A partiell gespalten und durch Saccharose-Gradienten-Zentrifugation (Maniatis et al., s.o.) fraktioniert. Fragmente mit einer Größe von 15-20 kb werden mit λ EMBL3-Armen ligiert (Maniatis et al., s.o.). Diese ligierte DNA wird in Phagenpartikel (Amersham) verpackt. Pro $\mu$g DNA werden 2 x $10^6$ rekombinante Phagen erhalten.

"Screenen" der Genbank nach OMPF-Sequenzen:

Rekombinante Phagen werden mit einer Dichte von 500 pfu auf einem NM539-Bakterienrasen ausplattiert. Phagenplaques werden nach der Methode von Benton und Davies (Science 196, 180-182, 1977) auf Nylon-Membranen übertragen. Durch Hybridisierung mit den Oligonuleotiden 5′ AAC/TC/TTA/GGCC/TGAC/TTTC/TATGAA 3′ und 5′ TCNGCA/GTTC/TTTCATA/GAA 3′, welche nach den Peptidsequenzen NLADFMK und NMKNAD synthetisiert wurden, werden aus 2500 rekombinanten Phagenplaquese 6 positive Kandidaten isoliert.

4

Sequenzanalyse des OMPF-Gens und seiner flankierenden Bereiche:

Von einem der isolierten Phagen wird eine Großkultur (1l) gezüchtet und daraus DNA präpariert (Maniatis et al., s.o.). Diese wird mit den Restriktionsenzymen Sall und Sau3A (partiell) gespalten. Die erhaltenen Restriktionsfragmente werden "shotgun" in pUC18 und pUC19 (Yanisch-Perron et al., Gene 33, 103-119, 1985) subkloniert. Aus hybridisierungspositiven Klonen wird Plasmid-DNA isoliert und diese nach weiterer Subklonierung, bzw. Exo III- und Exo VII-Verdauung (Yanisch-Perron et al., s.o.), nach der Methode von Chen und Seeburg (DNA 4, 165-170, 1985) sequenziert.

**Beispiel 3:**

**Expression von OMPF**

Da die Expression von OMPF in E. coli toxisch für die Bakterien ist, sofern das Strukturgen auf einem "medium copy"- (pBR322) oder "high copy"- (pUC-Plasmid) Vektor unter der Kontrolle des eigenen Promotors vorliegt, wird das Strukturgen zunächst unter die Kontrolle eines induzierbaren Promotors gebracht. Ein Teilfragment des OMPF-Gens, welches den Promotor und den 5′-terminalen Teil des Strukturgens enthält, wird als Sall-Restriktionsfragment in Sall-geschnittene M13mp19 doppelsträngige DNA (Yanisch-Perron, s.o.) ligiert, und damit nach Standardbedingungen (Hanahan, J. Mol. Biol. 166, 557-580, 1983) E. coli JM109 (Yanisch-Perron et al., s.o.) transformiert. Aus einer 5 ml Flüssigkultur transformierter Bakterien werden aus dem Überstand Phagen gewonnen und daraus wird einzelsträngige DNA präpariert. Nach der Methode von Newman et al. (Cell 42, 335-344, 1985) wird mit Hilfe eines Oligodesoxynucleotids eine gezielte Mutagenese an dieser einzelsträngigen DNA durchgeführt, um direkt vor dem ATG-Translationsinitiationscodon eine EcoRI-Restriktionsschnittstelle einzuführen, d.h. die Sequenz ATTTAACGGATG (Nucleotide 55-66 in Tabelle 1) wird in die Sequenz ATTGAATTCATG umgewandelt. Aus diesem neuen Konstrukt wird nun der 5′-terminale Teil des Strukturgens herausgeschnitten und unter die Kontrolle des induzierbaren tac-Promotors (de Boer al al., Proc. Natl. Acad. Sci. USA 78, 21 ff, 1983) gebracht. Zu diesem Zweck wird das EcoRI-Sall-Fragment in die EcoRI- und Sall-Schnittstellen des Vektors pKK223-3 (Pharmacia) ligiert. Dieses klonierte Zwischenprodukt wird mit Sall und PstI geschnitten und in diese Schnittstellen wird der restliche Teil des OMPF-Strukturgens inklusive Terminatorregion als Sall-PstI-Fragment eingebracht. Das OMPF-Strukturgen steht nun unter der Kontrolle des induzierbaren tac-Promotors. Durch Zugabe von IPTG (Isopropyl-$\beta$-D-thiogalactosid) zu der Kultur transformierter Zellen (JM105, Yanisch-Perron et al, s.o.), wird der tac-Promotor dereprimiert und damit die Expression von OMPF induziert.

**Beispiel 4:**

**Herstellung von Antiseren**

Gesunde Erwachsene, die in ihrer Krankheitsgeschichte keine Allergien, Diabetes, Immundefizienz-Krankheiten, Anämien oder Hautkrankheiten aufweisen, werden mit heterolog exprimiertem OMPF, bzw. Teilsequenzen davon, immunisiert. Die Vaccinierung erfolgt an den Tagen 1, 8, und 15. Drei Wochen nach der letzten Injektion wird den freiwilligen Kandidaten Blut entnommen und dieses auf Hepatitis B-Oberflächenantigen sowie auf HIV-Antigene getestet. Nur negativ reagierende Plasmaspenden werden gepoolt, unter kontrollierten sterilen Bedingungen fraktioniert und abgepackt.

**Beispiel 5:**

**Herstellung von monoklonalen Antikörpern**

Gereinigtes OMPF, bzw. OMPF-Teilpeptide davon, werden in Freunds Adjuvans oder Al(OH)$_3$ Balb/c-Mäusen intraperitoneal injiziert. Nach 6 Wochen wird mit gelöstem Antigen "geboostet". Der Antikörpertiter wird eine Woche später durch ELISA-Tests bestimmt. Bei unzureichender Immunreaktion erfolgen weitere Injektionen. 3 Tage vor der Zellfusion werden die Mäuse mit 10 $\mu$g des gelösten Antigens intravenös "geboostet".

Die Milzzellen werden nach Standardmethoden (nach Köhler und Milstein) mit NS1-Zellen fusioniert. Nach Selektion in HAT-Medium wachsen in den Mikronäpfen einzelne Kolonien aus, deren Kulturüberstände wiederum im ELISA auf Antikörper gegen OMPF getestet werden. Positive Kolonien werden subkloniert.

Die Antikörper werden aus Kulturüberständen und aus in Balb/c-Mäusen induzierten Asciten gewonnen, nach gängigen biochemischen Methoden gereinigt und charakterisiert.

## Tabelle

GCCACCCAAGTTGTGCGGTGATTGTTGGACAACTAACTGACCATCAAGATGGGGATTTAA

```
CGGATGAAACTGAAGAACACCTTAGGCGTTGTCATCGGCTCGCTGGTTGCCGCTTCGGCA
        M   K   L   K   N   T   L   G   V   V   I   G   S   L   V   A   A   S   A

ATGAACGCCTTCGCCCAGGGCCAGAACTCGGTAGAGATCGAAGCCTTCGGCAAGCGCTAC
 M   N   A   F   A   Q   G   Q   N   S   V   E   I   E   A   F   G   K   R   Y

TTCACCGACAGCGTTCGCAACATGAAGAACGCTGACCTGTACGGCGGCTCGATCGGCTAC
 F   T   D   S   V   R   N   M   K   N   A   D   L   Y   G   G   S   I   G   Y

TTCCTGACCGACGACGTCGAGCTGGCTCTGTCCTACGGTGAGTACCACGATGTTCGTGGC
 F   L   T   D   D   V   E   L   A   L   S   Y   G   E   Y   H   D   V   R   G

ACCTACGAAACCGGCAACAAGAAGGTCCATGGCAACCTGACCTCCCTGGACGCCATCTAC
 T   Y   E   T   G   N   K   K   V   H   G   N   L   T   S   L   D   A   I   Y

CACTTCGGTACCCCGGGCGTAGGTCTGCGTCCGTACGTGTCGGCTGGTCTGGCTCACCAG
 H   F   G   T   P   G   V   G   L   R   P   Y   V   S   A   G   L   A   H   Q

AACATCACCAACATCAACAGCGACAGCCAAGGCCGTCAGCAGATGACCATGGCCAACATC
 N   I   T   N   I   N   S   D   S   Q   G   R   Q   Q   M   T   M   A   N   I

GGCGCTGGTCTGAAGTACTACTTCACCGAGAACTTCTTCGCCAAGGCCAGCCTCGACGGC
 G   A   G   L   K   Y   Y   F   T   E   N   F   F   A   K   A   S   L   D   G

CAGTACGGCCTGGAGAAGCGTGACAACGGTCACCAGGGTGAGTGGATGGCTGGCCTGGGC
 Q   Y   G   L   E   K   R   D   N   G   H   Q   G   E   W   M   A   G   L   G

GTCGGCTTCAACTTCGGTGGTTCGAAAGCCGCTCCGGCTCCGGAACCGGTTGCCGACGTT
 V   G   F   N   F   G   G   S   K   A   A   P   A   P   E   P   V   A   D   V

TGCTCCGACTCCGACAACGACGGCGTCTGCGACAACGTCGACAAGTGCCCGGACACCCCG
 C   S   D   S   D   N   D   G   V   C   D   N   V   D   K   C   P   D   T   P

GCCAACGTCACCGTTGACGCCAACGGCTGCCCGGCTGTCGCCGAAGTCGTACGCGTACAG
 A   N   V   T   V   D   A   N   G   C   P   A   V   A   E   V   V   R   V   Q.

CTGGACGTGAAGTTCGACTTCGACAAGTCCAAGGTCAAAGAGAACAGCTACGCTGACATC
 L   D   V   K   F   D   F   D   K   S   K   V   K   E   N   S   Y   A   D   I

AAGAACCTGGCCGACTTCATGAAGCAGTACCCGTCCACTTCCACCACCGTTGAAGGTCAT
 K   N   L   A   D   F   M   K   Q   Y   P   S   T   S   T   T   V   E   G   H

ACCGACTCCGTCGGTACCGACGCTTACAACCAGAAGCTGTCCGAGCGTCGTGCCAACGCC
 T   D   S   V   G   T   D   A   Y   N   Q   K   L   S   E   R   R   A   N   A

GTTCGTGACGTACTGGTCAACGAGTACGGTGTGGAAGGTGGTCGCGTGAACGCTGTCGGT
 V   R   D   V   L   V   N   E   Y   G   V   E   G   G   R   V   N   A   V   G

TACGGCGAGTCCCGCCCGGTTGCCGACAACGCCACCGCTGAAGGCCGCGCTATCAACCGT
 Y   G   E   S   R   P   V   A   D   N   A   T   A   E   G   R   A   I   N   R

CGCGTTGAAGCCGAAGTAGAAGCCGAAGCCAAGTAATCGGCTGAGCCTTCAAAGAAAAAC
 R   V   E   A   E   V   E   A   E   A   K   *

CGGCCCAGGCCGGGTTTTTCTTTGCCTGGAAAAAGACCGCTCGTCAGGCGCTCAGGGAAA

CCGGTTGCGACACGATGCCGCGGGCCACTTCGCCGATCTGGGTCGACCTGCAG
```

6

EP 0 297 291 B1

**Patentansprüche**

**Patentansprüche für folgende Vertragsstaaten : AT, BE, CH, DE, FR, GB, HL, IT, LI, LU, SE**

1. Nukleotid, kodierend für das äußere Membranprotein F (OMPF) von Pseudomonas aeruginosa, mit der DNA (kodierender Strang) gemäß Tabelle oder für immunogene Sequenzen kodierende Teile davon.

2. Vektoren und DNA-Struktur, die das Nukleotid nach Anspruch 1 oder für immunogene Sequenzen kodierende Teile davon enthalten.

3. Pro- oder eukaryotische Zellen, die Vektoren oder DNA-Strukturen nach Anspruch 2 enthalten.

4. Verfahren zur Herstellung von OMPF oder immunogenen Teilsequenzen davon, dadurch gekennzeichnet, daß eine pro- oder eukaryotische Zelle mit Vektoren oder DNA-Strukturen nach Anspruch 2 transformiert und die genannte DNA exprimiert wird.

5. Verfahren zur Herstellung von OMPF oder immunogenen Teilsequenzen davon, dadurch gekennzeichnet, daß eine pro- oder eukaryotische Zelle nach Anspruch 3 kultiviert wird.

6. Verwendung von Antigenen, hergestellt nach einem Verfahren nach Anspruch 4 oder 5, zur Induzierung von Lymphozyten zur Produktion entsprechender monoklonaler Antikörper oder zum Testen von Lymphozyten auf die Produktion solcher Antikörper.

7. Verwendung von Antigenen, hergestellt nach einem Verfahren nach Anspruch 4 oder 5, zur Herstellung von Antiseren.

8. Diagnostikum, das das Nukleotid nach Anspruch 1 ganz oder teilweise oder ein davon abgeleitetes Nukleotid enthält.

9. Diagnostizierverfahren unter Verwendung eines Diagnostikums nach Anspruch 8.

**Patentansprüche für folgenden Vertragsstaat : ES**

1. Verfahren zur Herstellung von OMPF oder immunogenen Teilsequenzen, dadurch gekennzeichnet, daß eine pro- oder eukaryotische Zelle mit Vektoren oder DNA-Strukturen, die ein Nukleotid, kodierend für das äußere Membranprotein F (OMPF) von Pseudomonas aeruginosa, mit der DNA (kodierender Strang) gemäß Tabelle oder Teile davon, enthalten, transformiert und die genannte DNA exprimiert wird.

2. Verfahren zur Herstellung von OMPF oder immunogenen Teilsequenzen, dadurch gekennzeichnet, daß eine pro- oder eukaryotische Zelle, die mit Vektoren oder DNA-Strukturen, die ein Nukleotid, kodierend für das äußere Membranprotein F (OMPF) von Pseudomonas aeruginosa, mit der DNA (kodierender Strang) gemäß Tabelle oder Teile davon, enthalten, kultiviert wird.

3. Verfahren zur Herstellung einer transformierten pro- oder eukaryotischen Zelle, dadurch gekennzeichnet, daß eine pro- oder eukaryotische Zelle mit Vektoren oder DNA-Strukturen, die ein Nukleotid kodierend für das äußere Membranprotein F (OMPF) von Pseudomonas aeruginosa, mit der DNA (kodierender Strang) gemäß Tabelle oder für immunogene Sequenzen kodierende Teile davon, enthalten, transformiert wird.

4. Verwendung von Antigenen, hergestellt nach einem Verfahren nach Anspruch 1 oder 2, zur Induzierung von Lymphozyten zur Produktion entsprechender monoklonaler Antikörper oder zum Testen von Lymphozyten auf die Produktion solcher Antikörper.

5. Verwendung von Antigenen, hergestellt nach einem Verfahren nach Anspruch 1 oder 2, zur Herstellung von Antiseren.

6. Verfahren zur Herstellung eines Diagnostikums, dadurch gekennzeichnet, daß ein Nukleotid, kodierend für das äußere Membranprotein F (OMPF) von Pseudomonas aeruginosa, mit der DNA (kodierender

7

Strang) gemäß Tabelle oder Teile davon, verwendet wird.

7. Diagnostizierverfahren unter Verwendung eines Diagnostikums, das ein Nukleotid, kodierend für das äußere Membranprotein F (OMPF) von Pseudomonas aeruginosa, mit der DNA (kodierender Strang) gemäß Tabelle oder Teile davon, oder ein davon abgeleitetes Nukleotid enthält.

**Claims**
**Claims for the following Contracting States : AT, BE, CH, DE, FR, GB, IT, LI, LU, NL, SE**

1. A nucleotide coding for outer membrane protein F (OMPF) of Pseudomonas aeruginosa, having the DNA (coding strand) shown in the table or parts thereof which code for immunogenic sequences.

2. Vectors and DNA structure which contain the nucleotide as claimed in claim 1 or parts thereof which code for immunogenic sequences.

3. Pro- or eukaryotic cells which contain vectors or DNA structures as claimed in claim 2.

4. A process for the preparation of OMPF or immunogenic part-sequences thereof, which comprises transforming a pro- or eukaryotic cell with vectors or DNA structures as claimed in claim 2, and expressing the said DNA.

5. A process for the preparation of OMPF or immunogenic part-sequences thereof, which comprises cultivating a pro- or eukaryotic cell as claimed in claim 3.

6. The use of antigens prepared by a process as claimed in claim 4 or 5 for the induction of lymphocytes for the production of corresponding monoclonal antibodies or for testing lymphocytes for the production of such antibodies.

7. The use of antigens prepared by a process as claimed in claim 4 or 5 for the preparation of antisera.

8. A diagnostic aid which contains the nucleotide as claimed in claim 1 in whole or in part, or a nucleotide derived therefrom.

9. A diagnostic method which makes use of a diagnostic aid as claimed in claim 8.

**Claims for the following Contracting State : ES**

1. A process for the preparation of OMPF or immunogenic part-sequences, which comprises transforming a pro- or eukaryotic cell with vectors or DNA structures which contain a nucleotide coding for outer membrane protein F (OMPF) of Pseudomonas aeruginosa, having the DNA (coding strand) shown in the table or parts thereof, and expressing the said DNA.

2. A process for the preparation of OMPF or immunogenic part-sequences, which comprises cultivating a pro- or eukaryotic cell which is transformed with vectors or DNA structures which contain a nucleotide coding for outer membrane protein F (OMPF) of Pseudomonas aeruginosa, having the DNA (coding strand) shown in the table or parts thereof.

3. A process for the preparation of a transformed pro- or eukaryotic cell, which comprises transforming a pro- or eukaryotic cell with vectors or DNA structures which contain a nucleotide coding for outer membrane protein F (OMPF) of Pseudomonas aeruginosa, having the DNA (coding strand) shown in the table or parts thereof which code for immunogenic sequences.

4. The use of antigens prepared by a process as claimed in claim 1 or 2 for the induction of lymphocytes for the production of corresponding monoclonal antibodies or for testing lymphocytes for the production of such antibodies.

5. The use of antigens prepared by a process as claimed in claim 1 or 2 for the preparation of antisera.

EP 0 297 291 B1

6. A process for the preparation of a diagnostic aid, which comprises using a nucleotide coding for outer membrane protein F (OMPF) of Pseudomonas aeruginosa, having the DNA (coding strand) shown in the table or parts thereof.

7. A diagnostic method which makes use of a diagnostic aid which contains a nucleotide coding for outer membrane protein F (OMPF) of Pseudomonas aeruginosa, having the DNA (coding strand) shown in the table or parts thereof, or a nucleotide derived therefrom.

**Revendications**

**Revendications pour les Etats contractants suivants : AT, BE, CH, DE, FR, GB, IT, LI, LU, NL, SE**

1. Nucléotide, codant pour la protéine membranaire externe F (OMPF) de Pseudomonas aeruginosa, comportant l'ADN (brin codant) selon le Tableau ou des parties de cet ADN codantes pour les séquences immunogènes.

2. Vecteurs et structure d'ADN, qui contiennent le nucléotide de la revendication 1 ou des parties de ce nucléotide codantes pour les séquences immunogènes.

3. Cellules procaryotes et eucaryotes, qui contiennent des vecteurs ou des structures d'ADN selon la revendication 2.

4. Procédé de fabrication de OMPF ou de ses séquences immunogènes partielles, caractérisé en ce qu'on transforme une cellule procaryote ou eucaryote avec des vecteurs ou des structures d'ADN selon la revendication 2 et qu'on exprime ledit ADN.

5. Procédé de fabrication de OMPF ou de ses séquences immunogènes partielles, caractérisé en ce qu'on cultive une cellule procaryote ou eucaryote selon la revendication 3.

6. Utilisation d'antigènes, fabriqués d'après un procédé selon l'une des revendications 4 ou 5, pour stimuler la production par les lymphocytes des anticorps monoclonaux correspondants ou pour évaluer la capacité des lymphocytes à produire de tels anticorps.

7. Utilisation d'antigènes, fabriqués d'après un procédé selon l'une des revendications 4 ou 5, pour fabriquer des antisérums.

8. Agent de diagnostic, qui contient, intégralement ou partiellement, le nucléotide selon la revendication 1 ou un nucléotide qui en dérive.

9. Procédé de diagnostic mettant en oeuvre un agent de diagnostic selon la revendication 8.

**Revendications pour l'Etat contractant suivant : ES**

1. Procédé de fabrication d'une protéine membranaire externe F (OMPF) ou de séquences immunogènes partielles, caractérisé en ce qu'on transforme une cellule procaryote ou eucaryote avec des vecteurs ou des structures d'ADN qui contiennent un nucléotide codant pour l'OMPF de Pseudomonas aeruginosa, comportant l'ADN (brin codant) du Tableau ou des parties de cet ADN, et qu'on exprime ledit ADN.

2. Procédé de fabrication d'OMPF ou de séquences immunogènes partielles, caractérisé en ce qu'on cultive une cellule procaryote ou eucaryote, qui a été transformée avec des vecteurs ou des structures d'ADN qui contiennent un nucléotide codant pour l'OMPF de Pseudomonas aeruginosa, comportant l'ADN (brin codant) du Tableau ou des parties de cet ADN.

3. Procédé de fabrication d'une cellule procaryote ou eucaryote transformée, caractérisé en ce qu'on transforme une cellule procaryote ou eucaryote avec des vecteurs ou des structures d'ADN qui contiennent un nucléotide codant pour l'OMPF de Pseudomonas aeruginosa, comportant l'ADN (brin codant) du Tableau ou des parties de cet ADN codantes pour les séquences immunogènes.

9

**4.** Utilisation d'antigènes, fabriqués d'après un procédé selon une des revendications 1 ou 2, pour stimuler la production par les lymphocytes des anticorps monoclonaux correspondants ou pour évaluer la capacité des lymphocytes à produire de tels anticorps.

**5.** Utilisation d'antigènes, fabriqués d'après un procédé selon une des revendications 1 ou 2, pour fabriquer des antisérums.

**6.** Procédé de fabrication d'un agent de diagnostic, caractérisé en ce qu'on utilise un nucléotide codant pour l'OMPF de Pseudomonas aeruginosa, comportant l'ADN (brin codant) du Tableau ou des parties de cet ADN .

**7.** Procédé de diagnostic mettant en oeuvre un agent de diagnostic qui contient un nucléotide codant pour l'OMPF de Pseudomonas aeruginosa, comportant l'ADN (brin codant) du Tableau ou des parties de cet ADN, ou bien un nucléotide qui en dérive.